# EUROPEAN PATENT APPLICATION

(11) **EP 2 959 895 A1**
(43) Date of publication of application: **30.12.2015**
(21) Application number: 14174835.0
(22) Date of filing: 27.06.2014
(51) Int. Cl.: A61K 31/198, G01N 33/68

(54) **Compositions comprising Phenylalanine and Leucine for use in the diagnosis and treatment of neurological disorders and symptoms thereof**

(71) Applicant: Rheinisch-Westfälische Technische Hochschule Aachen (RWTH), 52062 Aachen (DE)
(72) Inventor: Zepf, Florian Daniel, 52076 Aachen-Kornelimünster (DE)
(74) Representative: Steinecke, Peter

(57) **Abstract**

Provided is a novel neurodietary supplement essentially consisting of the amino acids phenylalanine (Phe) and leucine (Leu), which is useful in the diagnosis and treatment of neurological, neuropsychiatric, or sleep disorders. Furthermore, a method of predicting the clinical response of a subject suffering from or being at risk to develop a neurological, neuropsychiatric, or sleep disorder to selective serotonin reuptake inhibitors (SSRI) and possibly other pharmacological agents using the novel neurodietary supplement is described.

## Description

### Field of the present invention

The present invention relates to the use of the specific combination of the amino acids phenylalanine (Phe; F) and leucine (Leu; L) in the diagnosis and/or treatment of neurological, neuropsychiatric, or sleep disorders. Furthermore, the present invention relates to the use of the amino acids Phe and Leu in a challenge test before, whilst or after such treatment. In addition, the present invention relates to pharmaceutical and diagnostic compositions as well as neurodietary supplements comprising Phe and Leu which are useful in the treatment of neurological or neuropsychatric disorders and/or as predictor of the subject's response to a pharmacological or psychotherapeutic treatment in a neurological, neuropsychatric, or sleep disorder.

### Background of the invention

The neurotransmitter serotonin (5-hydroxytryptamine (5-HT)) plays an essential role in a wide range of neuropsychiatric, behavioral, and sleep disorders. The treatment of such disorders with serotonergic drugs can be accompanied by serotonin syndrome (SS), a potentially life-threatening toxic state caused by an adverse drug reaction that leads to excessive central and peripheral serotonergic activity. This excessive serotonin-related hyperstimulation may be secondary to 1 standard therapeutic dose of a single agent, inadvertent interactions between various drugs, intentionally or unintentionally excessive use of particular drugs, deliberate self-harm, or recreational use of certain drugs; see Iqbal et al., in Annals of Clinical Psychiatry 24 (2012), 310-318 for review including a table for drug combinations associated with SS.

Thus, methods were developed to investigate the role of 5-HT in cognitive functions and neuropsychiatric disorders in such patient populations, and to determine whether a patient may be amenable to serotonergic drug treatment and at risk of developing SS, respectively. In this context, Acute Tryptophan Depletion (ATD) is a widely used dietary method to study the effects of serotonin (5-hydroxytryptamine (5-HT)) in cognition and behavior in mammals by leading to a short-term and reversible reduction of central nervous serotonin (5-HT) synthesis. As such, ATD-based studies have implicated 5-HT in numerous cognitive functions and have also advanced understanding of depression and affective disorders.

The neurotransmitter serotonin (5-HT) is not able to pass the Blood-Brain-Barrier (BBB) and is thus synthesized from its physiological precursor amino acid tryptophan (Trp) in the brain. Acute Tryptophan Depletion (ATD) is based on the principle that several amino acids including Trp use the same transport system; *i.e.* large neutral amino acid (LNAA) transport system (L-1) in the capillary cell plasma membrane to pass the Blood-Brain-Barrier (BBB). Thus, administration of a neurodietary supplement, which lacks Trp, the physiological amino acid precursor of 5-HT, diminishes the uptake of Trp into the brain. Since all the relevant amino acids in said neurodietary supplement use the same active large neutral amino acid (LNAA) transport system (L-1) in the capillary cell plasma membrane to pass the BBB, an increase in plasma concentrations of other amino acids achieved by their neurodietary administration impacts the transcapillary influx of Trp over the BBB, which follows the principles of facilitated diffusion.

The administration of an amino acid beverage without Trp causes competitive antagonism at the L-1 of the consumed competitive amino acids (CAAs) with endogenous Trp. Due to these competing amino acids the overall influx of Trp across the BBB is diminished. As a result of the decreased Trp uptake into the central nervous system (CNS) there is reduced substrate availability for tryptophan hydroxylase 2 (TPH2), the enzyme that limits the rate of synthesis of 5-HT in the CNS. Moreover, amino acid intake stimulates protein synthesis in the liver. This synthesis requires additional Trp from plasma reserves and contributes to 5-HT depletion, in addition to the passive diffusion of amino acids across the BBB.

The ATD-test protocols employed in the prior art comprised amino acid mixtures which included a high number of amino acids at high doses and are complicated to prepare and were often not well-tolerated; *i.e.* being associated with nausea, vomiting, headache and dizziness as described by Delgado et al., Arch. Gen. Psychiatry 47 (1990), 411-418.

Side effects related to the amino acid beverages used in tryptophan depletion methods that have been employed so far are one of the most serious disadvantages. The most frequently reported side effects are extensive nausea, vomiting, sweating, fast heart, shaking, dizziness, irritability, nausea, anxiety, depression, tension, headache and loss of appetite, to loss of concentration, tiredness and stomach ache; Badawy et al. in "Specificity of the acute Tryptophan and Tyrosine plus Phenylalanine depletion and loading tests I. Review of biochemical aspects and poor specificity" Int. J. Tryp. Research 3 (2010), 23-34. These side effects lead to drop-outs during challenge experiments and additionally interfere with behaviors that are being assessed.

Moreover, the currently employed amino acid compositions exhibit rather poor specificity as also mentioned by Badawy *et al.,* 2010. Specificity of the ATD-test formulation in particular relates to the rate of serotonin synthesis being decreased, and with no or only minor changes to the rate of dopamine or noradrenaline synthesis.

Therefore, there is still a need for neurodietary compositions lacking Trp which can be administered to mammals, both humans and animals, and which are well-tolerated without the side-effects mentioned above, so that they can be useful in a tryptophan depletion method for the study, diagnosis and/or treatment of the effects of central nervous serotonin metabolism. In addition, there is a need for an amino acid composition with a rather high specificity thus enabling a reliable diagnosis or evaluation of the impact of serotonin on the investigated neurological or neuropsychiatric disorders.

These technical problems are addressed by the embodiments characterized in the claims and described further below and illustrated in the Examples and Figures.

### Summary of the invention

In general, the present invention relates to a method of tryptophan (Trp; E) depletion in the brain by administering the two amino acids phenylalanine (Phe; F) and leucine (Leu; L) and, based thereon, provides methods for diagnosing, treating and preventing a disease and/or disorder associated with the synthesis and level of central nervous serotonin (5-HT). More particularly, the present invention relates to the use of phenylalanine (Phe; F) and leucine (Leu; L) in the diagnosis and/or treatment of a neurological, neuropsychiatric disorder, or sleep disorders and to neurodietary supplements essentially consisting of Phe and Leu.

The present invention also relates generally to a method of predicting the response of a subject to a serotonergic drug or tryptophan comprising:
(a) administering to the subject the amino acids phenylalanine (Phe; F) and leucine (Leu; L) or a neurodietary supplement essentially consisting of phenylalanine (Phe; F) and leucine (Leu; L); and
(b) determining the level of tryptophan (Trp), 2-(5-Hydroxy-1H-indol-3-yl)acetic acid (5-HIAA)), kynurenine, and/or serotonin in a sample of a body fluid from the subject, preferably a sample from serum, plasma, urine or the CNS, wherein the sample taken from the CNS is the cerebrospinal fluid;
wherein a decrease in the level of Trp, 5-HIAA, kynurenine, and/or serotonin compared to a sample taken from the subject before step (a) is indicative for a serotonergic responder. In particular, a decreased level is indicative for a serotonergic responder with regard to administration of the above-mentioned amino acids. The method of the present invention may be applied both to healthy subjects for example in order or determine whether they are receptive to over the counter drugs and homeopathic remedies as well as to patients suffering from or being at risk to develop a neurological or neuropsychiatric disorder in order to determine their clinical response to serotonergic drugs such as selective serotonin reuptake inhibitors (SSRI) compared to non-serotonergic antidepressant drugs, a placebo, and/or control condition. In both cases, the method of the present invention provides information on the subject's and patient's treatment response or non-response, and/or side-effects and adverse reactions with regard to drug treatment such as SSRI administration.

The amino acids for use in the diagnosis and/or treatment of a neurological or neuropsychiatric disorder or in the method according to the present invention are designed to be administered at a dose of 0.1 to 1 g/kg per body weight, preferably the dose for Phe is about [±25%] 0.32 g/kg per body weight and/or the dose for Leu is about [±25%] 0.3 g/kg per body weight.

In addition, or alternatively the amino acids for use in the treatment of a neurological or neuropsychiatric disorder or in the method according to the present invention may be designed to be administered in the form of a pharmaceutical composition, a dietary or neurodietary supplement or an amino acid enriched meal.

Furthermore, the pharmaceutical composition, dietary, or neurodietary supplement presented herein may be packaged in a packaging material and identified in print, in or on the packaging material, for use in the treatment of a neurological, neuropsychiatric disease or disorder, or sleep disorders.

In one embodiment of the present invention said dietary supplement is preferably in the form of an enteral protein-based meal. The dietary supplement may further comprise one or more other ingredients, preferably carbohydrates and/or fats to compose a daily life type of enteral and/or parenteral nutrition.

In a further embodiment, the neurodietary supplement, pharmaceutical composition, or dietary supplement does not substantially comprise isoleucine (Ile), valine (Val), and/or tyrosine (Tyr). In one embodiment, the present invention provides a neurodietary supplement essentially consisting of Phe and Leu preferably in the form of an enteral protein-based meal, which is substantially free of tryptophan (Trp).

The neurological or neuropsychiatric disorder to be diagnosed and/or treated in accordance with the present invention may be selected from the group consisting of mania, hypomania, bipolar disorders (in particular bipolar I and bipolar II), schizophrenia, schizoaffective disorders, depressive disorders, dysthymia, eating disorders (in particular anorexia nervosa, bulimia nervosa, eating disorders not otherwise specified), obsessive compulsive disorders, attention disorders (such as ADHD and related disorders), conduct disorders, disruptive mood dysregulation disorder, somatoform disorders, chronic fatigue syndrome, emotional disorders, autism spectrum disorders, addiction disorders, serotonin syndrome and/or childhood and adult onset dystonia.

Furthermore, the amino acid mixture of the present invention can be used in the analysis or treatment of sleep disorders, for example in order to support and/or promote the propensity for sleep, and/or facilitate falling asleep in insomnia. In one embodiment, the method utilizing the amino acid mixture as describe herein can be used before the administration of a drug associated with sleep, *e.g.* tryptophan, to test whether the drug support and/or promote the propensity for sleep, and/or facilitate falling asleep.

In one embodiment the present invention, the neurological, neuropsychiatric, or sleep disorder to be diagnosed and/or treated arises secondary to drug treatment, for example whereby the drug is a psychoactive drug.

In one embodiment the psychoactive drug is selected from the group consisting of selective serotonin reuptake inhibitors (SSRIs) Citalopram (Cipramil®), Escitalopram (Cipralex®), Fluoxetine (Fluctin®), Fluvoxamine (Fevarin®), Paroxetine (Serotax®), and/or Sertraline (Zoloft®); selective serotonin-noradrenaline-reuptake-inhibitors (SNRIs) Duloxetine (Cymbalta®, Yentreve®), Venlafaxine (Efexor®, Trevilor®), and/or Milnaciprane (Joncia, Ixel, Salvella, Dalcipran, Tivanyl, Toledomi); and/or monoamine oxidase inhibitors (MAO-inhibitors) Rasagilin, Selegilin, Tranylcypromin, Phenelzin, Moclobemid, Harmalin, Lazabemid; and or neuroleptic agents (Amisulpirid, Chlorprothixen, Clozapine, Flupentixol, Fluohenazin, Haloperidol, Levomepromazin, Melperone, Olanzapine, Perazine, Perphenazine, Pimozide, Pipamperone, Qutiapine, Risperidone); and/or psychostimulants (Amphetamine/Amphetamine derivates , Methylphenidate , Modafinil, Pemolin, Atomoxetine); antiepileptic agents and benzodiazepines (Carbamazepine, Clonazepam, Diazepam, Ethosuximid, Felbamat, Gabapentin, Lamotrigin, Lorazepam, Mesuximid, Oxcarbazepin, Phenobarbital, Phenytoin, Primidon, Sultiam, Tiagabin, Valproate, Vigabatrin), anxiolytic agents (Alprazolam, Buspiron, Clobazam, Dikaliumchlorazepat, Diphenhydramin, Doxylamin, Flunitrazepam, Hydroxyzin, Lorazepam, Lormetazepam, Opipramol, Temazepam, Zopiclon, Zolpidem); and/or agents to treat enuresis/encopresis (Desmopressin, Imipramin, Oxybutynin, Propiverin, Tolterodin), and/or adrenergic alpha-2-receptor antagonists (Clonidin, Guanfacin); and/or beta-receptor blockers (Propanolol), Buspiron, Sekretin; and/or opiate-receptor antagonists (Naltrexone); mood-stabilizers; L-tryptophan.

Further, the present invention relates to a neurodietary supplement essentially consisting of the amino acids phenylalanine (Phe) and leucine (Leu).

The present invention further relates to a combination preparation comprising the amino acids phenylalanine (Phe) and leucine (Leu) and a psychoactive drug such as one described above, wherein the amino acids phenylalanine (Phe) and leucine (Leu) are designed to be administered in conjunction with the drug either concomitantly or sequentially. Preferably, the amino acids are administered at a dose as indicated above.

In a further aspect, the present invention relates to a psychoactive drug such as any one referred to above for use in the diagnosis and/or treatment of a subject suffering from or being at risk to develop a neurological or neuropsychiatric disorder, wherein said drug is
(a) a serotonergic drug and designed to be administered to a subject diagnosed in accordance with the method of predicting the clinical response of a subject suffering or being at risk to develop a neurological or neuropsychiatric disorder to selective serotonin uptake, as defined in the present invention, as serotonergic responder; or
(b) a non-serotonergic drug and designed to be administered to a subject diagnosed in accordance with the method of predicting the clinical response of a subject suffering or being at risk to develop a neurological or neuropsychiatric disorder to selective serotonin uptake, as defined in the present invention, not to be a serotonergic responder.

The present invention further relates to a composition essentially consisting of Phe and Leu for challenging a response in the level of Trp, 5-HIAA, kynurenine, and/or serotonin in serum, plasma, urine, and/or the CNS, preferably in the cerebrospinal fluid in a subject as a predictor of the subject's response to a pharmacological or psychotherapeutic treatment in a neurological, neuropsychatric, or sleep disorder.

In a still further embodiment of the present invention, the pharmaceutical, dietary and neurodietary composition as defined above is used in the treatment and/or prophylaxis and/or study of certain serotonin related mental disorders such as aggression, depression, sexual disorders, to affect exercise capacity, or the like. In addition, the present invention relates to a method for affecting the serotonin metabolism in mammals, in particular humans, by administering to said mammal an effective amount of a neurodietrary supplement essentially consisting of phenylalanine (Phe) and leucine (Leu).

Further embodiments of the present invention will be apparent from the description and Examples that follow.

### Definitions

Within the meaning of the present invention, the terms "Acute Tryptophan Depletion" (ATD) and "Rapid Tryptophan Depletion" (RTD) are used synonymously.

Unless otherwise indicated, as used herein, the term "amino acids" includes naturally occurring amino acids (*e.g.,* L-phenylalanine, and L-leucine), their isomers, including optical isomers (*e.g.,* dextrorotatory (D-), levorotatory (L-), or mixtures thereof (DL-)), and analogs thereof. Mixtures of naturally occurring aromatic amino acids, isomers, and analogs are also contemplated. Preferably, all amino acids employed are L-, that is, the naturally occurring optical isomer configuration.

The term "neuropsychiatric disorder/s" refers to brain-function related diseases, disorders or dysfunctions that can cause psychiatric symptoms (such as defined in classification systems such as ICD (International Statistical Classification of Diseases and Related Health Problems) and DSM (Diagnostic and Statistical Manual of Mental Disorders) in their latest revisions). Non-limiting examples of neuropsychiatric disorders include depressive disorders, such as bipolar depression, etc., schizophrenia, PTSD and other anxiety disorders, autism, ADHD, and the like.

The term "neurological disorder" generally relates to a disorder of the brain or central nervous system, disease or dysfunction of/in the central, peripheral, and/or autonomic nervous system, involving and/or including impaired function of brain cells, impaired function of nerve cells and/or impaired transmission of neurological impulses all causing neurological or other somatic symptoms. "Neurological disorders" also include but are not limited to tumors of the brain, brain-related tumors, and tumors associated with tryptophan.

The term "sleep disorder" is utilized herein as a general term for sleep problems. Sleep disorders mean every alteration of the "normal" sleep, *i.e.* difficulties falling and/or staying asleep. Non-limiting examples of sleep disorders include oversleeping, insomnia, narcolepsy, REM sleep behavior disorder (RBD), circadian rhythm sleep disorder, hypersomnia, sleep-related eating disorders, shift work sleep disorder, and/or disruptive sleep disorders such as parasomnias.

As used herein, the term "treating/treatment of a neurological or neuropsychiatric disorder" comprises counteracting a neurological or neuropsychiatric disorder. Hence, complete recovery is not necessary as long as a patient's status is improved in comparison to his/her state before the initiation of the treatment.

The terms "psychoactive drug", "psychopharmaceutical", or "psychotropic" are used identically. If not specifically indicated otherwise, the term "psychoactive drug" is usedhereinto specifically refer to synthetic or natural chemical substances which cross the Blood-Brain-Barrier (BBB) and/or act upon the central nervous system (CNS) affecting at least one brain function, resulting in alterations in at least mood, consciousness, cognition, behavior and perception.

The term "overnight fasting" within the meaning of the present invention refers to the condition in which a mammal, *i.e.* human or animal, does not consume food, beverage or any kind of therapeutic or non-therapeutic substance, except for water for a period of at least 6 to 10 hours.

### Detailed description of the invention

The present invention generally relates to certain amino acids for use in the diagnosis and/or treatment of a neurological, neuropsychiatric, or sleep disorder and for abnormal movement disorders and for use in a serotonin/tryptophan challenge test for predicting the clinical response of a subject suffering from or being at risk to develop neurological or neuropsychiatric disorder due to selective serotonin reuptake inhibitors (SSRI).

As already mentioned, the current amino acid beverages administered to achieve an ATD effect are bound to severe side effects which can lead to the abortion of the challenge. Furthermore, they comprise several amino acids at high doses which resulting in a variety of biochemical mechanisms in the body leading to confusing and difficult evaluation of the related therapeutic response. The presence of several amino acids at different concentrations makes it difficult to find an appropriate dose for the challenge test.

In order to solve the problems mentioned above, the ATD-test Moja-De was developed.

The standard neurodietary challenge procedure ATD Moja-De includes a dosage per 10 kg of body weight of L-phenylalanine (1.32 g), L-leucine (1.32 g), L-isoleucine (0.84 g), L-methionine (0.5 g), L-valine (0.96 g), L-threonin (0.6 g) and L-lysine (0.96 g).

The ATD-test Moja-De was better tolerated and could also be administered also to pediatric populations. However, still a considerable number of side effects can be observed not only in children but also in adults even when the neurodietary challenge procedure ATD Moja-De is applied. Moreover, the neurodietary supplement employed in the ATD-test Moja-De still comprises several amino acids at different concentrations so that the individual dosing as well as the body-weight adapted dosing to the supplement is quite complicated. As a further consequence of the presence of a larger number of amino acids, several parameters have to be determined or quantified for monitoring purposes thus making the monitoring of the challenge test difficult, expensive and susceptible to errors.

Thus, there is a need for an ATD test protocol including an amino acid beverage with a possibly less amount of amino acids.

The specific combination of the two amino acids phenylalanine (Phe) and leucine (Leu) for use as a neurodietary supplement essentially consisting of these two amino acids overcomes the above-mentioned problems. As will be explained further below and described in the Examples, it has been surprisingly found that administration of the two amino acids Phe and Leu at a concentration of only about twice as much as in the ATD-test Moja-De but in sum substantially below the total amount of amino acids (6.2 g/10 kg body weight for Phe and Leu in accordance with the present invention versus 6.5 g/10 kg body weight for the amino acid mixture in accordance with the ATD-test Moja-De) nevertheless results in an improved acute tryptophan depletion thereby overcoming the disadvantages of the current beverages, as mentioned above.

Thanks to the findings of the present invention an improved ATD test protocol is provided, which achieves an effective reduction of the 5-HT synthesis in order to investigate or predict the clinical response of a subject suffering from or being at risk to develop a neurological or neuropsychiatric disorder to the therapy applied as well as designed to be used as a challenge test prior to a therapy with a psychoactive drug in order to predict whether the symptoms are serotonin-related.

Furthermore, due to reduction of the number of different amino acids and in addition lower amount, a neurodietary supplement is provided, which reduces the side effects related to high serotonin levels by achieving an acute tryptophan depletion at high magnitude.

In one embodiment, the present invention relates to the use of the two amino acids Phenylalanine (Phe) and Leucine (Leu) as a challenge test before, whilst or after treatment of neurological or neuropsychiatric disorders. Preferably, the two amino acids are designed to be administered for reducing the influx of tryptophan (Trp) via the blood-brain barrier (BBB) and/or the level and synthesis rate of serotonin (5-HT) in the central nervous system (CNS) via competitive antagonism with other amino acids as well as use of stimulated protein synthesis in the liver. This effect is based on the principle that the administration of Phe and Leu can lead to a subsequent decline in Trp concentrations in plasma and serum and thus lead to a decreased precursor supply for serotonin synthesis in terms of Trp.

Experiments performed within the scope of the present invention have surprisingly demonstrated that Acute Tryptophan Depletion (ATD) based on the administration of the two amino acids phenylalanine (Phe) and leucine (Leu) leads to a higher depletion magnitude in terms of a decreased Trp influx across the BBB compared to a standard ATD method; *e.g.* the ATD Moja-De protocol. Surprisingly, this effect was observed although the ATD method according to the present invention consisted of two amino acids; *i.e.* phenylalanine (Phe) and leucine (Leu) compared to the standard ATD methods used in the prior art, which employ for instance in case of ATD Moja-De seven different amino acids. Even more surprising was the fact that the overall dose of the amino acids phenylalanine (Phe) and leucine (Leu) was much more lower than the doses used ATD methods in the prior art. Additionally, it was surprisingly found that the two amino acids phenylalanine (Phe) and leucine (Leu) were well-tolerated by humans. Thus, in one embodiment of the present invention relates to a neurodietary supplement essentially consisting of Phe and Leu.

One further advantage of the present invention is that the pharmaceutical composition, dietary or neurodietary supplement comprising the two amino acids phenylalanine (Phe) and leucine (Leu) are much easier to produce due to the reduced amount of amino acid content. A further advantage owned by the present invention is that the ATD-method based on the two amino acids phenylalanine (Phe) and leucine (Leu) enables a facilitated monitoring of relevant blood parameters, such as amino acid concentrations, since their number is also reduced in terms of their measurement in the blood.

The main problem associated with patient tolerance of the ATD-beverages up to date is the unpleasant taste and large amounts of amino acids; *e.g.* ATD method Moja-De including seven amino acids at high doses. Thus, the reduction of the amount; *i.e.* two amino acids instead of seven and the dose as well as the absence of unpleasantly tasting amino acids such as methionine, cysteine and arginine, contribute to an improved acceptance of the ATD composition according to the present invention as well as a better patient compliance.

In accordance with the present invention, the treatment of neurological or neuropsychiatric disorders by administering the amino acids phenylalanine (Phe) and leucine (Leu) at doses based on a body weight adapted dosing regimen. Thus, the ATD method based on the present invention as well as the pharmaceutical composition, dietary/neurodietary supplement comprising/essentially consisting of the two amino acids phenylalanine (Phe) and leucine (Leu) are applicable not only to adults but also to paediatric populations opening at the same time a novel and inventive treatment opportunity.

The amino acids for use in the diagnosis and/or treatment of a neurological, neuropsychiatric, or sleep disorder according to the present invention are designed to be administered at a dose of 0.1 to 1 g/kg per body weight, see also *infra,* preferably the dose for Phe is about [±25%] 0.32 g/kg per body weight and/or the dose for Leu is about [±25%] 0.3 g/kg per body weight.

According to the present invention, the amino acids for use in the diagnosis and/or treatment of a neurological or neuropsychiatric disorder according to the present invention are designed such that said amino acids are administered in the form of an amino acid enriched meal, aqueous suspension, pharmaceutical composition or a dietary supplement, preferably wherein said meal, composition and supplement, respectively, may further include carbohydrates, fat and several other ingredients other than amino acids as such. In another embodiment, the pharmaceutical composition or dietary supplement essentially consists of the amino acids phenylalanine (Phe) and leucine (Leu).

Therefore the present invention further relates to dietary or neurodietary supplements in solid, liquid, or semiliquid form, characterized in that said dietary or neurodietary supplement comprises the amino acids Phe and Leu according to the present invention. The inventors have additionally noted that, dietary supplement according to the present invention can be prepared by combining it with non-toxic acceptable edible carriers to make either immediate release or slow release formulations, as is well known in the art. Such edible carriers may be either solid or liquid such as, for example, corn starch, lactose, sucrose, soy flakes, peanut oil, olive oil, sesame oil, and propylene glycol. The dosage forms may also contain preserving, stabilizing, wetting or emulsifying agents, solution promoters, etc. They may also contain other therapeutically valuable substances.

In accordance with the present invention, the amino acids Phe and Leu, for example in form of a pharmaceutical composition or (neuro)dietary supplement are designed to be administered at a dose sufficient to achieve the desired therapeutic and/or diagnostic effect, *i.e.* reducing the influx of tryptophan (Trp) via the blood-brain barrier (BBB) and/or the level and synthesis rate of serotonin (5-HT) in the central nervous system (CNS). Testing and validation of the effective amounts and compositions of the two amino acids Phe and Leu can be performed in appropriate animal models known in the art; see, *e.g.,* the experiments for dietary manipulation of serotonergic and dopaminergic function in C57BL/6J mice with amino acid depletion mixtures described in Sanchez et al., Neural. Transm. 121 (2014), 153-162; and the experiments on the effects of ATD on brain serotonin function and concentrations of dopamine and norepinephrine in C57BL/6J and BALB/cJ Mice described in Biskup et al., PLoS ONE 7(5): e35916. doi:10.1371/journal.pone.0035916. Furthermore, in accordance with the present invention it is preferred that the amino acids Phe and Leu are present in any of the compositions such as pharmaceutical composition, (neuro)dietary supplement, meal, etc. and are administered, respectively, in equal amounts per weight. Typical doses range from about 0.01 to 10 g/kg per body weight, preferably about 0.1 to 1 g/kg per body weight, more preferably about 0.2 to 0.4 g/kg per body weight and most preferably at a dose for Phe at about [±25%] 0.32 g/kg per body weight and/or the dose for Leu is about [±25%] 0.3 g/kg per body weight.

In one embodiment the amino acids phenylalanine (Phe) and leucine (Leu) for use in the diagnosis and/or treatment of neurological or neuropsychiatric disorders in accordance with the present invention are designed to be administered in form of a pharmaceutical composition, or a dietary supplement or a neurodietary supplement once daily.

In a further embodiment the amino acids phenylalanine (Phe) and leucine (Leu) are designed to be administered in form of a pharmaceutical composition, or a dietary supplement or a neurodietary supplement several times daily.

In another embodiment, the amino acids Phe and Leu for use in the diagnosis and/or treatment of neurological or neuropsychiatric disorders in accordance with the present invention are designed to be administered in form of a pharmaceutical composition or a dietary supplement or a neurodietary supplement once daily or several times daily immediately following an overnight fast. The overnight fast can be conducted once or several times.

As mentioned, it could be surprisingly shown that the specific combination of the two amino acids Phe and Leu as a neurodietary supplement at a concentration of only about twice as much as in the ATD-test Moja-De but in sum substantially below the total amount of amino acids (6.2 g versus 6.5 g/10 kg body weight) is sufficient to achieve acute tryptophan depletion. Accordingly, the use of the specific combination of the two amino acids Phe and Leu and neurodietary supplement of the present invention, respectively, may be distinguished from amino acid mixtures and beverages of the prior art by substantially the absence of any other amino acid or being present in amount less than in the prior art mixtures and beverages. Thus, in one embodiment amino acids in the neurodietary supplement or any other composition of the present invention other than Phe and Leu, in particular any other branched amino acid, methionine, tyrosine, tryptophan and/or lysine, if present at all, either alone or in combination is/are present in amount of less than in said prior art amino acid mixtures, for example less than 1 mg/10 kg body weight, preferably less than 0.5 mg/10 kg body weight and more preferably less than 0.1 mg/10 kg body weight.

In one embodiment of the pharmaceutical composition or dietary supplement the present invention does not substantially comprise isoleucine (Ile), valine (Val), and/or tyrosine (Tyr). In addition, or alternatively the pharmaceutical composition or the dietary supplement the present invention is lacking tryptophan (Trp).

In principle, any neurological, neuropsychiatric, or sleep disorder dependent from, associated with, accompanied by serotonin and/or induced by serotonergic drug treatment may be treated or diagnosed in accordance with the present invention. For example, the disorder may be selected from the group consisting of mania, hypomania, bipolar disorders (in particular bipolar I and bipolar II), schizophrenia, schizoaffective disorders, depressive disorders, dysthymia, eating disorders (in particular anorexia nervosa, bulimia nervosa, eating disorders not otherwise specified), obsessive compulsive disorders, attention disorders (such as ADHD and related disorders), conduct disorders, disruptive mood dysregulation disorder, somatoform disorders, chronic fatigue syndrome, emotional disorders, autism spectrum disorders, addiction disorders, serotonin syndrome and/or childhood and adult onset dystonia, as well as oversleeping, insomnia, narcolepsy, REM sleep behavior disorder (RBD), circadian rhythm sleep disorder, hypersomnia, sleep-related eating disorders, shift work sleep disorder, and/or disruptive sleep disorders such as parasomnias.

As discussed in the hereinabove and in the background section, any of the disorders may also arise secondary to drug treatment, for example during or after a drug treatment, in particular if the drug is a psychoactive serotonergic drug. Thus, in one embodiment, the present invention relates to the use of the amino acids Phe and Leu in the treatment or diagnosis of a neurological or neuropsychiatric disorder, wherein the disorder arises secondary to drug treatment and wherein said drug is a psychoactive drug.

Examples for a psychoactive drug include but are limited to the group consisting of selective serotonin reuptake inhibitors (SSRIs) Citalopram (Cipramil®), Escitalopram (Cipralex®), Fluoxetine (Fluctin®), Fluvoxamine (Fevarin®), Paroxetine (Serotax®), and/or Sertraline (Zoloft®); selective serotonin-noradrenaline-reuptake-inhibitors (SNRIs) Duloxetine (Cymbalta®, Yentreve®), Venlafaxine (Efexor®, Trevilor®), and/or Milnaciprane (Joncia, Ixel, Salvella, Dalcipran, Tivanyl, Toledomi); and/or monoamine oxidase inhibitors (MAO-inhibitors) Rasagilin, Selegilin, Tranylcypromin, Phenelzin, Moclobemid, Harmalin, Lazabemid; and or neuroleptic agents (Amisulpirid, Chlorprothixen, Clozapine, Flupentixol, Fluohenazin, Haloperidol, Levomepromazin, Melperone, Olanzapine, Perazine, Perphenazine, Pimozide, Pipamperone, Qutiapine, Risperidone); and/or psychostimulants (Amphetamine/Amphetamine derivates, Methylphenidate, Modafinil, Pemolin, Atomoxetine); antiepileptic agents and benzodiazepines (Carbamazepine, Clonazepam, Diazepam, Ethosuximid, Felbamat, Gabapentin, Lamotrigin, Lorazepam, Mesuximid, Oxcarbazepin, Phenobarbital, Phenytoin, Primidon, Sultiam, Tiagabin, Valproate, Vigabatrin), anxiolytic agents (Alprazolam, Buspiron, Clobazam, Dikaliumchlorazepat, Diphenhydramin, Doxylamin, Flunitrazepam, Hydroxyzin, Lorazepam, Lormetazepam, Opipramol, Temazepam, Zopiclon, Zolpidem,); and/or agents to treat enuresis/encopresis (Desmopressin, Imipramin, Oxybutynin, Propiverin, Tolterodin), and/or adrenergic alpha-2-receptor antagonists (Clonidin, Guanfacin); and/or beta-receptor blockers (Propanolol), Buspiron, Sekretin; and/or opiate-receptor antagonists (Naltrexone); mood-stabilizers; L-tryptophan.

In one embodiment the mood-stabiliser is selected from the group consisting of lithium, valproic acid, divalproex sodium; sodium valproate; lamotrigine; carbamazepine.

In one embodiment the psychoactive drug is selected from the group consisting of atypical antipsychotics; Amisulpride, Aripiprazole, Asenapine, Blonanserin, Clozapine, Iloperidone, Lurasidone, Melperone, Olanzapine, Paliperidone, Quetiapine, Risperidone, Serindole, Sulpiride, Ziprasidone, Zotepine.

In one further embodiment the psychoactive drug is selected from the group consisting of stimulants (psychostimulants); substituted phenethylamine (amphetamine), methylphenidate, dexmethylphenidate, dextroamphetamine, mixed amphetamine salts, dextromethamphetaime, and lixdexamfetamine.

In view of the above, the present invention also relates to a combination preparation comprising the amino acids Phe and Leu and a psychoactive drug as defined hereinbefore, wherein the amino acids Phe and Leu are designed to be administered in conjunction with the drug either concomitantly or sequentially. Moreover, here the dose of the two amino acids may range from about 0.1 to 1 g/kg per body weight, preferably wherein the amino acids are designed to be administered in conjunction with the drug either concomitantly or sequentially, preferably once daily immediately following an overnight fasting. Preferably, dose for Phe is about [±25%] 0.32 g/kg per body weight and/or the dose for Leu is about [±25%] 0.3 g/kg per body weight.

The total amount of the amino acids to be administered, based on molecular weight and body weight as indicated above daily, administered in one dose or subdivided into multiple sub-doses, *e.g.* 3 or 4 sub-doses, spread throughout the day. The amino acids can be administered in the form of various pharmaceutical preparations such as tablets, capsules, flavored bars, suspensions, emulsions, beverages, etc. Liquid formulations can be prepared by mixing amino acid powder with various liquids such as water and juices such as orange juice, etc. Since the dosage is best optimized for an individual patient, a suggested starting dosage is about [±25%] 0.32 for Phe and about [±25%] 0.3 g/kg for Leu per body weight per day followed by regular patient monitoring, optionally with increasing or decreasing the dosages in increments of 50 mg/kg/day as presence or absence, increase or decrease, of symptoms is evaluated, thereby reaching the lowest effective dose.

The administration of an amino acid beverage without Trp causes the competitive antagonism at the L-1 of the consumed competitive amino acids (CAAs) with endogenous Trp. Due to these amino acids the overall influx of Trp across the BBB is diminished. As a result of the decreased Trp uptake into the central nervous system (CNS) there is reduced substrate availability for tryptophan hydroxylase 2 (TPH2), the enzyme that limits the rate of synthesis of 5-HT in the CNS. Moreover, amino acid intake stimulates protein synthesis in the liver. This synthesis requires additional Trp from plasma reserves and contributes to 5-HT depletion, in addition to the passive diffusion of amino acids across the BBB (Zepf 2012; Principles of rapid tryptophan depletion and its use in neuropsychiatric disorders. In: D'Mello JPF (ed) Amino acids in human nutrition and health, Cabi Publishing, Oxfordshire, 418-426). In this context, as mentioned above the ATD-test is also used in the assessment of whether or not a patient suffering from neurological disorder, in particular psychiatric disorders may be amenable to serotonergic drug treatment; see, *e.g.,* Delgado et al. in Psychopharmacol. Bull. 27 (1991), 321-330 for the principle of this test illustrated by rapid serotonin depletion as a provocative challenge test for patients with major depression.

Therefore, in a further aspect, the present invention relates to a corresponding serotonin/tryptophan challenge assay, *i.e.* a method of predicting the clinical response of a subject suffering from or being at risk to develop a neurological, neuropsychiatric, or sleep disorder to selective serotonin reuptake inhibitors (SSRI) compared to non-serotonergic antidepressant drugs, a placebo, and/or control condition comprising:
(a) administering to the subject the amino acids Phe and Leu or a neurodietary supplement essentially consisting of Phe and Leu; and
(b) determining the level of tryptophan (Trp), 2-(5-Hydroxy-1H-indol-3-yl)acetic acid (5-HIAA), kynurenine, and/or serotonin in a sample of a body fluid from the subject, preferably a sample from serum, plasma, urine or the CNS, wherein the sample taken from CNS is the cerebrospinal fluid;
wherein a decreased level of Trp, 5-HIAA, kynurenine, and/or serotonin compared to a sample taken from the subject before step (a) is indicative for a serotonergic responder.

In particular, as described above, a decrease in levels of Trp, 5-HIAA, kynurenine, and/or serotonin compared to a sample, see *supra,* is indicative for the treatment response or non-response, and/or side-effects and adverse reactions, for example with regard to an SSRI administration.

In this context, it is to be understood that the level of Trp, 5-HIAA, kynurenine, and/or serotonin as well as competing branched changed amino acids (BCAAs) may be directly determined, *e.g.* using Trp, 5-HIAA, kynurenine, and/or serotonin ELISA assays or enzymatic tests, see also the Examples, or indirectly, for example by determining the level of compounds which correlate with the level of Trp, 5-HIAA, kynurenine, and/or serotonin. For example, evidence for elevations of serotonin anti-idiotypic antibodies in patients with a neurological disorder has been reported; see, *e.g.,* Coplan et al. in Neuropsychopharmacology 20 (1999), 386-391, reporting that plasma anti-serotonin and serotonin-anti-idiotypic antibodies are elevated in panic disorder. In addition, autoantibodies to serotonin in serum of patients with psychiatric disorders have been described; see Schotta et al., Psychiatry Research 121 (2003), 51-57. Thus, in one embodiment of the present invention the administration of the amino acids Phe and Leu can be designed to be dependent from and/or in relation to the occurrence of autoantibodies against serotonin, dopamine, kynurenine, GABA, glutamate, etc.

In addition, in one embodiment of the method of the present invention, the increased levels of autoantibodies to Trp, 5-HIAA, kynurenine, and/or serotonin compared to a sample taken from the subject before step (a) is indicative for a serotonergic responder and favorable outcome for a treatment with SSRI. As described above, an altered level of an autoantibody can be used for the identification of the treatment response or non-response, and/or side-effects and adverse reactions with regard to an SSRI administration.

The present invention further relates to a composition essentially consisting of Phe and Leu for challenging a response in the level of Trp, 5-HIAA, kynurenine and/or serotonin in serum, plasma, urine and/or the CNS, preferably in the cerebrospinal fluid in a subject as a predictor of the subject's response to a pharmacological or psychotherapeutic treatment in a neurological or neuropsychiatric disorder. The ATD effect reduced Trp influx over the BBB as achieved within the scope of the present invention is discovered to be much higher than the conventional methods; for instance the ATD Moja-De protocol. Considering the fact that the conventional ATD methods; *e.g.* ATD Moja-De, include a higher amount of amino acids at higher doses, this effect is unexpected and surprising. The LNAAs have different affinities to the common L-1 transporter which is mathematically calculated by employing the so-called Michaelis constant (Kₘ). Basically, Kₘ is indicative of the specific substrate concentration needed to achieve half maximal rate of turn-over of an enzyme. Thus, the lower the substrate's Kₘ value the higher its affinity to the related enzyme.

Pardridge and Oldendorf (Biochim Biophys Acta. 401(1975), 128-136) investigated Michealis-Menten kinetics of blood brain barrier (BBB) transport for fourteen amino acids in anesthetized rats, demonstrating the lowest Kₘ values for phenylalanine (0.12 µmol/ml) and leucine (0.15 µmol/ml). Their higher affinity to the common L-1 transporter compared to the other LNAAs causes the best displacement of Tryptophan (Trp) at the common transport and therefore leads to a greater reduction of Trp influx across the BBB. In contrast, ATD Moja-De, containing a mixture of seven different amino acids with partly lower Kₘ values leads to a less effective competition against Trp, thus resulting in a smaller Trp depletion magnitude when compared to the ATD method employed within the scope of other present invention; *i.e.* by administering the amino acids phenylalanine and leucine in the absence of further amino acids. The inventors of the present invention have discovered that the competitive mechanism of the amino acids enables a higher effectiveness of Trp depletion after ATD achieved by Phe and Leu administration, despite the simultaneous reduction of the overall amino acid dose.

Thus, the present invention is based on the effect of the higher depletion magnitude of Trp influx rates across the BBB in healthy adults following the administration of the ATD protocol according to the present invention which includes the administration of Phe and Leu in comparison to the standard protocol ATD Moja-De, comprising seven amino acids at higher doses. The inventors of the present invention were thus able to achieve, by administering only the relevant amino acids dosed; *i.e.* only the two LNAAs Phe and Leu, a simplified blood monitoring due to reduced components administered to the patients.

Further, the compositions and supplements within the scope of the present invention are much better tolerated and exhibit much less adverse effects since these include much less amino acids at much reduced doses. By means of the present invention, it is possible to administer a composition to achieve an ATD effect by employing a much more reduced overall amount of amino acids. Thus, the present invention provides also a method for investigating the physiological and behavioral effects of reduced central nervous 5-HT synthesis related to neurological, neuropsychiatric, or sleep disorders.

Furthermore, the present invention relates a diagnostic composition and kit, which comprises one or more components necessary and/or useful for performing the serotonin/tryptophan challenge assay of the present invention. The kit may contain, for example amino acids Phe Leu, and/or or BCAAs, components of an enzyme-linked immunosorbent assay (ELISA) and enzymatic tests such as primary and secondary antibodies which are optionally linked to a detectable enzyme or label for the detection and/or means for detection and optionally calculation of sample levels of Phe, Tyr, Trp, any of branched-chained amino acids (BCAA; Leu, Ile, Val), 5-HIAA, kynurenine and/or serotonin. In addition, or alternatively the kit may contain antibodies against auto-antibodies against 5-HIAA, kynurenine and/or serotonin or against any other protein or small molecule which presence or level correlates with the level of 5-HIAA, kynurenine and/or serotonin in a body fluid of a patient, preferably blood after challenge with the amino acid combination of the present invention. The kit may optionally further comprise reference and/or control samples and/or values as well as instructions for use in a challenge assay and method. In a preferred embodiment, the kit of the present invention comprises and/or is used in combination with any one of the above described neurodietary supplement, the combination preparation or composition of the present invention. In one embodiment, the kit for use in accordance with the present invention comprises means for an immunoassay, preferably for performing the method of the present in an automatic assay format such as described in international application WO 2012/065912. In particular, the direct and indirect immunological determination of 5-HIAA, kynurenine and/or serotonin as well as L-tryptophan can be performed by way of adaptation of the experiments described in Examples 1 to 4 in WO 2012/065912 A1. Accordingly, the kit for use in accordance with the present invention may comprise derivatization reagents L-phenylalanine (MW = 165), serotonin (MW = 176), or L- tryptophan (MW = 204) as well as other serotonin precursors and metabolites. As discussed above, in accordance with the method of the present invention patients being diagnosed to suffer from a neurological or neuropsychiatric disorder prior to medication can be assessed for whether or not the respective patient is amenable to a serotonergic psychoactive drug and/or is as risk of developing serotonin syndrome. Depending on the outcome of this assessment either a serotonergic or non-serotonergic psychoactive drug may be prescribed. Thus, the specific amino acid combination of Phe and Leu in accordance with the present invention as described in its various embodiments hereinabove is particularly useful as a predictive test for personalized antidepressant treatment.

Accordingly, in a further aspect the present application relates to a psychoactive drug as defined hereinabove for use in the treatment of a subject suffering from or being at risk to develop a neurological, neuropsychiatric, or sleep disorder, wherein the drug is
(a) a serotonergic drug and designed to be administered to a subject diagnosed in accordance with the method of the present invention to be a serotonergic responder; or
(b) a non-serotonergic drug and designed to be administered to a subject diagnosed in accordance with the method of the present invention not to be serotonergic responder.

For example, in one embodiment the subject may be a patient suffering from a bipolar disorder or depression and, dependent for the diagnosis in accordance with the method of the present invention may be treated with a serotonergic psychoactive drug. Likewise, a subject suffering from a sleep disorder, dependent for the diagnosis in accordance with the method of the present invention may be prescribed a serotonergic soporific drug. In a preferred embodiment, the drug comprises L-tryptophan as available from ratiopharm or from STADAvita sold under the trademark KALMA®.

In a further embodiment, the amino acids Phe and Leu, and/or the compositions mentioned above can be applied in the diagnosis and/or treatment of tumors of/in the central nervous system, in particular tumors of the brain. In particular, in several studies it was shown that the use of radioactively marked amino acids, such as O-(2-[¹⁸F]fluoroethyl)-1-tyrosine (FET) and [¹¹C]-Methionin (MET), can improve diagnostic assessment of several tumors by positron-emission-tomography (PET), see *e.g.* Tovi et al., Acta Radiol. 31 (1990), 417-429; Ribom et al., J Neuroonkology, 71(2005), 325-332; Hamacher et al., Appl. Radiat. Isot. 57 (2002), 853-856; Langen et al., Nucl Med Biol. 33 (2006), 287-294. Without being bound by theory the amino acids Phe and Leu can lead to a further improvement of the FET-PET diagnosis due to their Trp depletion effect. In particular, tumors, specifically malignant tumors, have an increase in the uptake of amino acids. The administration of the amino acid mix Phe and Leu which leads to a Trp depletion and therefore to a decreased availability of Trp in the brain and accordingly in the tumor cells, would allow a higher uptake of other amino acids in the tumor cell including marked amino acids. As a consequence the tumor would better stained and a more clear prediction of the tumor would be capable. Likewise, the uptake of antitumor agents can be enhanced due to the decreased Trp, in particular in the brain, as consequence of the Phe/Leu induced Trp depletion.

These and other embodiments are disclosed and encompassed by the description and examples of the present invention. Further literature concerning any one of the materials, methods, uses, and compounds to be employed in accordance with the present invention may be retrieved from public libraries and databases, using for example electronic devices. For example the public database "Medline" may be utilized, which is hosted by the National Center for Biotechnology Information (NCBI) and/or the National Library of Medicine at the National Institutes of Health (NLM.NIH). Further databases and web addresses, such as those of the European Bioinformatics Institute (EBI), which is part of the European Molecular Biology Laboratory (EMBL) are known to the person skilled in the art and can also be obtained using internet-based search engines. An overview of patent information in biotechnology and a survey of relevant sources of patent information useful for retrospective searching and for current awareness is given in Berks, TIBTECH 12 (1994), 352-364.

Although the above identified diagnosis and/or treatment as well as methods described herein are preferably applied in humans, they also can be performed and/or tested in animals, in particular laboratory animals.

The above disclosure generally describes the present invention. Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2. Several documents are cited throughout the text of this specification. Full bibliographic citations may be found at the end of the specification immediately preceding the claims. The contents of all cited references (including literature references, issued patents, published patent applications as cited throughout this application including the background section and manufacturer's specifications, instructions, etc.) are hereby expressly incorporated by reference; however, there is no admission that any document cited is indeed prior art as to the present invention.

A more complete understanding can be obtained by reference to the following specific examples, which are provided herein for purposes of illustration only and are not intended to limit the scope of the invention.

### EXAMPLES

### Example 1:

In principle, acute tryptophan depletion in accordance with body weight and influx of amino acids across the blood-brain barrier can be assessed as described in Dingerkus et al., J. Neural. Transm. 119 (2012), 1037-1045. In a placebo-controlled, double blind study employed in healthy adult subjects (50% females and 50% males) an enhanced ATD effect was observed when the amino acids Phenylalanine (Phe) and Leucine (Leu) were administered immediately after an overnight fasting period in comparison to the ATD Moja-De protocol (Stadler et al., Hum. Psychopharmacol Clin Exp 23(2008), 43-51., Biskup et al., PLoS One. 7(5) (2012), e35916, Dingerkus et al., J Neural Transm. 119(9) (2012), 1037-1045).

The subjects were at ages of 18 and 40 with a mean age of about 30 to 35 years. All subjects were healthy adults; *i.e.* who did not suffer from any physical or psychiatric disorders. The exclusion criteria involved an IQ lower than 85, smoking, any current medication except of oral contraceptives, drug abuse, pregnancy, psycho-organic syndromes, schizophrenia, affective disorders as well as neurological and somatic diseases such as migraine, asthma, diabetes, metabolic or hormonal disorders, allergies or obesity. The mean BMI (Body-Mass-Index) of the whole sample was about between 20 to 25 kg/m².

The amino acid beverages consisting essentially of phenylalanine (Phe) and leucine (Leu) were administered to the subjects always at approximately the same time to avoid metabolic influences and fluctuations in blood values. The subjects were instructed to abstain from food with high protein content starting at 8:00 pm the night before the sampling and they were not allowed to have any beverages other than water; *i.e.* overnight fasting. The subjects received a standardized low protein breakfast immediately following the administration of the amino acids beverages.

The following additional amino acid compositions were produced as comparison:
BAL: Balanced Amino Acid Load; control amino acid composition including additionally tryptophan (Trp).
nBAL = "New Balanced Amino Acid Load"; control amino acid mixture including additionally tryptophan (Trp)

The standard neurodietary challenge procedure ATD Moja-De (RTD: Rapid Tryptophan Depletion-Test/Acute Tryptophan Depletion (ATD)-Test Moja-De) included a dosage per 10 kg of body weight of L-phenylalanine (1.342 g), L-leucine (1.342 g), L-isoleucine (0.84 g), L-methionine (0.5 g), L-valine (0.96 g), L-threonine (0.6 g) and L-lysin (0.96 g). The amino acid mixture developed with the meaning of the present invention (Phe/Leu) was also dosed per 10 g of body weight, only consisting of L-phenylalanine (3.2 g) and L-leucine (3.0 g).

Blood samples were drawn from patients after an overnight fasting (base line; T0) and after each hour (T1, T2, T3, T4, T5, T6) after the intake of ATD as described above (ATD_{Phe/Leu} and ATD Moja-De) as well as after intake of the control conditions. For Trp influx calculations plasma levels of phenylalanine (Phe), tyrosine (Tyr) and tryptophan (Trp) were measured using enzyme-linked immunosorbent assay kits (ELISA) (Immundiagnostik AG, Bensheim, Germany).

The Tryptophan (Trp) influx across the BBB was calculated as described by Pardridge WM in "Brain metabolism: a perspective from the blood-brain-barrier"; Physiol. Rev. (1983) 63: 1481-1535 as well as by Smith et al. in "Kinetics of neutral amino acid transport across the blood-brain-barrier"; J. Neurochem. 49 (1987), 1651-1658.

PANAS (Positive and Negative Affect Schedule) containing 10 items for positive (PA) and 10 items for negative effect (NA) as described by Watson et al. in "Development and validation of brief measures of positive and negative effect: the PANAS scales" J. Pers. Soc. Psychol. 54 (1988), 1063-1070 was utilised to assess the extent to which depletion influenced the subjects' mood state.

A questionnaire was developed in order to evaluate the tolerability of the amino acid challenge according to the present invention in comparison to the standard ATD Moja-De protocol. Relevant items were rated from 0 (disagree) to 3 (strongly agree). The common side effects known from the ATD Moja-De method or other known ATD methods of the prior art such as headache, nausea, vomiting, dizziness and sweating were covered by the somatic complaints score. A similar questionnaire was also developed for the evaluation of the taste of the amino acid supplement according to the present invention.

The influence of different amino acid supplements on Trp influx across the BBB, on tolerance; *i.e.* somatic-complaints score, hunger-score and awakeness-score, and on the PANAS evaluation over time was investigated using repeated measurements analyses variance (rmANOVAs) with treatment as between-subject factor, time as within-subject factor and gender as a covariate.

Human data demonstrated clear advantages of the neurodietary supplement according to the present invention (Phe/Leu) over the composition employed in the ATD Moja-De protocol. No significant differences in tryptophan (Trp) influx were present at base line (TO) when comparing ATD_{Phe/Leu} and ATD Moja-De. Maximum decrease in Trp influx four hours after the intake was 11.4% higher for ATD_{Phe/Leu} when compared to ATD Moja-De. Further, it was observed that ATD_{Phe/Leu} leads to an increase in Trp influx of approximately 5.1 % compared to the baseline (TO) from time point T4 to T5, followed by a significant increase from T5 to T6. Gender differences between males and females were not observed.

### Example 2:

The neurodietary supplement according to the present invention was also investigated in C57 mice. The concentrations were calculated 2.5 hours after ingestion of the amino acid composition according to the present invention (Phe/Leu) every 30 minutes; *i.e.* said composition comprising phenylalanine (Phe) at a dose of 3.2 g/10kg and leucine (Leu) at a dose of 3 g/10 kg. Further, the following compositions were used in the treatment as comparison:
BAL: Balanced Amino Acid Load; control amino acid composition including additionally tryptophan (Trp).
RTD: Rapid Tryptophan Depletion-Test / Acute Tryptophan Depletion (ATD)-Test Moja-De comprising phenylalanine (1.32 g/10kg), isoleucine (0.84 g/10kg), valine (0.96 g/10kg), leucine (1.32 g/10kg), threonine (0.6 g/10kg), lysine (0.96 g/10kg), methionine (0.5 g/10kg).

Further amino acid mixture: Amino acid mixture comprising phenylalanine (3 g/10kg), leucine (2.8 g/10kg) and isoleucine (2.5g/10kg), thereby the total amount of the amino acids being higher as in the neurodietary supplement according to the present invention.

The alterations in concentrations of 5-HIAA, serotonin and tryptophan in serum as well as in different brain regions of the mouse; *i.e.* hippocampus, amygdala, caudate nucleus, nucleus accumbens and front cortex were assessed. The results demonstrated that depending on the brain region the neurodietary supplement according to the present invention (Phe/Leu) overall lowered serotonin and tryptophan in different brain regions at different levels, although the former included a much less amount of and also fewer amino acids when compared to the (ATD)-Test Moja-De. The animal experiments clearly demonstrated that the neurodietary supplement (Phe/Leu) according to the present invention lowered serotonin, tryptophan and 5-HIAA in different regions of the brain at different levels.

Based on the theory underlying the present invention and current evidence, the specific combination of the two amino acids Phe and Leu appears to be an advantageous alternative to the common ATD protocols that are currently available when investigating the physiological and behavioral effects of reduced central nervous 5-HT synthesis in translational research and important neurodietary supplement for personalized medicine in the treatment of neurological, psychiatric, and sleep disorders.

## Claims

1. Amino acids phenylalanine (Phe) and leucine (Leu) for use in the diagnosis and/or treatment of a neurological, neuropsychiatric, or sleep disorder.

2. The amino acids for use in accordance with claim 1, wherein the amino acids are designed to be administered at a dose of 0.1 to 1 g/kg per body weight.

3. The amino acids for use in accordance with claim 1 or 2, wherein the dose for Phe is about 0.32 g/kg per body weight and/or the dose for Leu is about 0.3 g/kg per body weight.

4. The amino acids for use according to any one of claims 1 to 3, wherein the amino acids are administered in the form of an amino acid enriched meal, a tablet, a capsule, a beverage or an aqueous suspension.

5. The amino acids for use according to any one of claims 1 to 3, wherein the amino acids are administered in the form of a pharmaceutical composition or a dietary supplement.

6. The amino acids for use in accordance with claim 5, wherein the pharmaceutical composition or a dietary supplement does not substantially comprise isoleucine (Ile), valine (Val), and/or tyrosine (Tyr).

7. The amino acids for use in accordance with any one of claims 1 to 6, wherein the disorder is selected from the group consisting of mania, hypomania, bipolar disorders (in particular bipolar I and bipolar II), schizophrenia, schizoaffective disorders, depressive disorders, dysthymia, eating disorders (in particular anorexia nervosa, bulimia nervosa, eating disorders not otherwise specified), obsessive compulsive disorders, attention disorders (such as ADHD and related disorders), conduct disorders, disruptive mood dysregulation disorder, somatoform disorders, chronic fatigue syndrome, emotional disorders, autism spectrum disorders, addiction disorders, serotonin syndrome and/or childhood and adult onset dystonia, oversleeping, insomnia, narcolepsy, REM sleep behavior disorder (RBD), circadian rhythm sleep disorder, hypersomnia, sleep-related eating disorders, shift work sleep disorder, and/or disruptive sleep disorders.

8. The amino acids for use in accordance with any one of claims 1 to 7, wherein the disorder arises secondary to drug treatment.

9. The amino acids for use in accordance with claim 8, wherein the drug is a psychoactive drug, preferably wherein the psychoactive drugs is selected from the group consisting of selective serotonin reuptake inhibitors (SSRIs) Citalopram (Cipramil®), Escitalopram (Cipralex®),Fluoxetine (Fluctin®), Fluvoxamine (Fevarin®), Paroxetine (Serotax®), and/or Sertraline (Zoloft®); selective serotonin-noradrenaline-reuptake-inhibitors (SNRIs) Duloxetine (Cymbalta®, Yentreve®), Venlafaxine (Efexor®, Trevilor®), and/or Milnaciprane (Joncia, Ixel, Salvella, Dalcipran, Tivanyl, Toledomi); and/or monoamine oxidase inhibitors (MAO-inhibitors) Rasagiline, Selegiline, Tranylcypromin, Phenelzine, Moclobemid, Harmaline, Lazabemid; and/or neuroleptic agents (Amisulpirid, Chlorprothixen, Clozapine, Flupentixol, Fluohenazine, Haloperidol, Levomepromazine, Melperone, Olanzapine, Perazine, Perphenazine, Pimozide, Pipamperone, Qutiapine, Risperidone); and/or psychostimulants (Amphetamine/Amphetamine derivates, Methylphenidate, Modafinil, Pemoline, Atomoxetine); antiepileptic agents and benzodiazepines (Carbamazepine, Clonazepam, Diazepam, Ethosuximid, Felbamat, Gabapentin, Lamotrigin, Lorazepam, Mesuximid, Oxcarbazepin, Phenobarbital, Phenytoin, Primidon, Sultiam, Tiagabin, Valproate, Vigabatrin), anxiolytic agents (Alprazolam, Buspiron, Clobazam, Dikaliumchlorazepat, Diphenhydramin, Doxylamin, Flunitrazepam, Hydroxyzin, Lorazepam, Lormetazepam, Opipramol, Temazepam, Zopiclon, Zolpidem,); and/or agents to treat enuresis/encopresis (Desmopressin, Imipramin, Oxybutynin, Propiverin, Tolterodin), and/or adrenergic alpha-2-receptor antagonists (Clonidin, Guanfacin); and/or beta-receptor blockers (Propanolol), Buspiron, Sekretin; and/or opiate-receptor antagonists (Naltrexone); mood-stabilizer, L-tryptophan.

10. A neurodietary supplement essentially consisting of Phe and Leu.

11. A combination preparation comprising the amino acids as defined in any one of claims 1 to 6 and a psychoactive drug as defined in claim 9 or, wherein the amino acids are designed to be administered in conjunction with the drug either concomitantly or sequentially.

12. A method of predicting whether a subject is responsive to a serotonergic drug or tryptophan comprising:
(a) administering to the subject the amino acids as defined in any one of claims 1 to 6 or the neurodietary supplement of claim 10; and
(b) determining the level of tryptophan (Trp), 2-(5-Hydroxy-1H-indol-3-yl)acetic acid (5-HIAA)), kynurenine, and/or serotonin in a sample of a body fluid from the subject, preferably a sample from serum, plasma, urine or the CNS, wherein the sample taken from the CNS is preferably cerebrospinal fluid;
wherein a decreased level of Trp, 5-HIAA, kynurenine, and/or serotonin compared to a sample taken from the subject before step (a) is indicative for a serotonergic responder.

13. A composition essentially consisting of Phe and Leu for challenging a response in the level of Trp, 5-HIAA, kynurenine and/or serotonin in serum, plasma, urine and/or the CNS (*e.g.* cerebrospinal fluid) in a subject as a predictor of the subject's response to a pharmacological or psychotherapeutic treatment in a neurological, neuropsychatric, or sleep disorder.

14. A kit for use in the method of claim 12 or for use in combination with the neurodietary supplement of claim 10, the combination preparation of claim 11 or the composition of claim 13, the kit comprising amino acids Phe and/or Leu, components of an enzyme-linked immunosorbent assay (ELISA) such primary and secondary antibodies which are optionally linked to a detectable enzyme or label for the detection and optionally calculation of sample levels of Phe, Tyr, Trp, any of branched-chained amino acids (BCAA; Leu, Ile, Val), 5-HIAA, kynurenine and/or serotonin; optionally further comprising reference/control samples and/or values as well as instructions for use.

15. A psychoactive drug as defined in claim 9 for use in the treatment of a subject suffering from or being at risk to develop a neurological, neuropsychiatric, or sleep disorder, wherein the drug is
(a) a serotonergic drug and designed to be administered to a subject diagnosed in accordance with the method of claim 12 as serotonergic responder; or
(b) a non-serotonergic drug and designed to be administered to a subject diagnosed in accordance with the method of claim 12 not to be serotonergic responder.
